# EUROPEAN PATENT SPECIFICATION

(11) **EP 1 217 072 B1**
(45) Date of publication and mention of the grant of the patent: **22.07.2009**
(21) Application number: 01128498.1
(22) Date of filing: 07.12.2001
(51) Int. Cl.: C12N 15/66, C12N 15/67, C12N 5/16, C12P 21/02, A01K 67/00, C12N 15/64, C12N 15/79, C12N 5/10, A01K 67/027

(54) **Method for the preparation of an expression vector for the production of therapeutic proteins in transgenic animals**
Verfahren für die Herstellung eines Expressionsvektors zur Produktion von therapeutischen Proteinen in transgenen Tieren
Methode pour la préparation d'un vecteur d'expression de protéines thérapeutiques dans des animaux transgéniques

(30) Priority: 07.12.2000 CZ 20004569
(43) Date of publication of application: 26.06.2002
(73) Proprietor: Biopharm, Výzkumný ústav biofarmacie a Veterinárních léciv a.s., 254 49 Jílové u Prahy (CZ)
(72) Inventor: Mikus, Tomas, 140 00 Praha 4 (CZ); Maly, Petr, 140 00 Praha 4 (CZ)
(74) Representative: PATENTSERVIS Praha, a.s.

(56) References cited:
- SCHNEIDER S ET AL: "An epitope tagged mammalian/prokaryotic expression vector with positive selection of cloned inserts." GENE. NETHERLANDS 15 SEP 1997, vol. 197, no. 1-2, 15 September 1997 (1997-09-15), pages 337-341, XP002203455 ISSN: 0378-1119
- DATABASE MEDLINE [Online] July 1998 (1998-07) AGUIRRE A ET AL: "Expression of human erythropoietin transgenes and of the endogenous WAP gene in the mammary gland of transgenic rabbits during gestation and lactation." Database accession no. NLM9859219 XP001084634 & TRANSGENIC RESEARCH. ENGLAND JUL 1998, vol. 7, no. 4, July 1998 (1998-07), pages 311-317, ISSN: 0962-8819
- DATABASE MEDLINE [Online] 1996 MASSOUD M ET AL: "The deleterious effects of human erythropoietin gene driven by the rabbit whey acidic protein gene promoter in transgenic rabbits." Database accession no. NLM8987107 XP001084633 & REPRODUCTION, NUTRITION, DEVELOPMENT. FRANCE 1996, vol. 36, no. 5, 1996, pages 555-563, ISSN: 0926-5287
- MIKUS T ET AL: "Expression of human erythropoietin gene in the mammary gland of a transgenic mouse." FOLIA BIOLOGICA (PRAGUE), vol. 47, no. 6, 2001, pages 187-195, XP001083841 ISSN: 0015-5500

### SEQUENCE LISTING

<110> Biopharm Vyzkummy ustav biofarmacie a veterinarnic
<120> Method of the preparation of the expression vector for production of therapeutic proteins in transgenic animals
<130> 91046m3
<140> 01128498.1
   <141> 2001-12-07
<150> CZ PV 2000-4569
   <151> 2000-12-07
<160> 24
<170> PatentIn Ver. 2.1
<210> 1
   <211> 32
   <212> DNA
   <213> Artificial Sequence
<220>
   <223> Description of Artificial Sequence: oligomer primer
<400> -1 cgggatcctc atgtgccccg agcccagctc tt 32
<210> 2
   <211> 32
   <212> DNA
   <213> Artificial Sequence
<220>
   <223> Description of Artificial Sequence: oligomer primer
<400> 2 cgggatccgg gttccagata gcgcatggcg ac 32
<210> 3
   <211> 24
   <212> DNA
   <213> Artificial Sequence
<220>
   <223> Description of Artificial Sequence: oligomer primer
<400> 3 tgtgacagcc gagtcctgca gagg 24
<210> 4
   <211> 24
   <212> DNA
   <213> Artificial Sequence
<220>
   <223> Description of Artificial Sequence: oligomer primer
<400> 4 caagctgcag tgttcagcac agcc 24
<210> 5
   <211> 30
   <212> DNA
   <213> Artificial Sequence
<220>
   <223> Description of Artificial Sequence: oligomer primer
<400> 5 ccaggaagct gcgaggagct ctgtgcctga 30
<210> 6
   <211> 30
   <212> DNA
   <213> Artificial Sequence
<220>
   <223> Description of Artificial Sequence: oligomer primer
<400> 6 cgcatggcgg ccgctggtag gctggtggtg 30
<210> 7
   <211> 39
   <212> DNA
   <213> Artificial Sequence
<220>
   <223> Description of Artificial Sequence: oligomer primer
<400> 7 aagaatgcgg ccgccatggg ggtgcacggt gagtactcg 39
<210> 8
   <211> 24
   <212> DNA
   <213> Artificial Sequence
<220>
   <223> Description of Artificial Sequence: oligomer primer
<400> 8 caagctgcag tgttcagcac agcc 24
<210> 9
   <211> 20
   <212> DNA
   <213> Artificial Sequence
<220>
   <223> Description of Artificial Sequence: primer
<400> 9 taccacctgc caccatgcgc 20
<210> 10
   <211> 20
   <212> DNA
   <213> Artificial Sequence
<220>
   <223> Description of Artificial Sequence: oligonukleotide
<400> 10 ggccaggcgc ggagatgggg 20
<210> 11
   <211> 13
   <212> DNA
   <213> Artificial Sequence
<220>
   <223> Description of Artificial Sequence:optimized translational Kozak consensus
<400> 11 gccgccrcca tgg 13
<210> 12
   <211> 20
   <212> DNA
   <213> Artificial Sequence
<220>
   <223> Description of Artificial Sequence: Translational consensus WAP-EPO with NotI modification
<400> 12 taccagcggc cgccatgggg 20
<210> 13
   <211> 240
   <212> DNA
   <213> Artificial Sequence
<220>
   <223> Description of Artificial Sequence: Kozak consensus in gene for WAP
<400> 13
   tttgaaacca tgaccgcagc cgttcctcca acctggcctg acctctccac gtgtccaagg 60
   aggaagcccc ctggcccagt tgaggcctcg ccaacctggc acccctccag gctcctcctc 120
   ctgctccaac ctttaaatgc atcccggggc cccagaacac catccgacac ctgcctgctg 180
   cccaccacca gcctaccacc tgccaccatg cgctgtctca tcagcctggc cctcggcctg 240
<210> 14
   <211> 30
   <212> DNA
   <213> Artificial Sequence
<220>
   <223> Description of Artificial Sequence: primer
<400> 14 gtggtggtcg gatggtcgcc ggcggtacgc 30
<210> 15
   <211> 39
   <212> DNA
   <213> Artificial Sequence
<220>
   <223> Description of Artificial Sequence: primer
<400> 15 aagaatgcgg ccgccatggg ggtgcacggt gagtactcg 39
<210> 16
   <211> 240
   <212> DNA
   <213> Artificial Sequence
<220>
   <223> Description of Artificial Sequence: Kozakm consensus in gene for EPO
<400> 16
   gctgagggac cccggccagg cgcggagatg ggggtgcacg gtgagtactc gcgggctggg 60
   cgctcccgcc cgcccgggtc cctgtttgag cggggattta gcgccccggc tattggccag 120
   gaggtggctg ggttcaagga ccggcgactt gtcaaggacc ccggaagggg gaggggggtg 180
   gggcagcctc cacgtgccag cggggacttg ggggagtcct tggggatggc aaaaacctga 240
<210> 17
   <211> 60
   <212> DNA
   <213> Artificial Sequence
<220>
   <223> Description of Artificial Sequence: Resulting Kozak consensus modified in hybrid gene rWAP-hEPO with NotI restriction site
<400> 17 cccaccacca gcctaccagc ggccgccatg ggggtgcacg gtgagtactc gcgggctggg 60
<210> 18
   <211> 588
   <212> DNA
   <213> Artificial Sequence
<220>
   <223> Description of Artificial Sequence: probe 587 bp WAP ex2/ex3
<400> 18
   cgggatcctc atgtgccccg agcccagctc ttccgaggag acgctctgcc tcagtgacaa 60
   cgactgtctc ggcagcaccg tgtgctgtcc cagcgccgcc ggcggctcct gcagaacccc 120
   catcatcggt aacgtagcca cactgcaggc ctctccggaa gcccacacac ctgccccatg 180
   gcgcagtctc tctgggcccc atccacctgc cccgaggcct ctgtgccacc ccacaggtcc 240
   ctgagggctc caggatgccc cagtgctgcg ggaggtcctg cggtgagacc agcaagaggg 300
   aggccacaga gacccagctg acctcagggg tcccccggcg ctcaacttgt ctcagtgggg 360
   tcttgcgggt caggtctggg ggggcccatg ttacagggtg tgaccagaaa aggcctgtct 420
   ctccccagtc cctaccccca aggctggccg ctgcccctgg gtgcaggcgc caatgctgtc 480
   ccagttgtgt gaggagctga gcgactgtgc caacgacatc gagtgcaggg gcgacaagaa 540
   gtgctgcttc agccgctgcg ccatgcgcta tctggaaccc ggatcccg                588
<210> 19
   <211> 837
   <212> DNA
   <213> Artificial Sequence
<220>
   <223> Description of Artificial Sequence: probe 836pbp WAP 1 ex/p
<400> 19
   ccaggaagct gcgaggagct ctgtgcctga ggccggagca gggtcgctgg ctggacaggg 60
   ctgtggcccc cagccatcct gccctggggt ctccgcagtc cccatggccc cttccctgtc 120
   tgggtcctgg gggggcgggt gcaggaacta cacggccagc agcacatccg cccctgccct 180
   gtggcacctg ctcccctggc acagggcaca ggagggcctt ccgagaagag accttctgtc 240
   ccctcgcccc tccacagtcg gcaagcctgc actggggtcc ccagggcagg ggcccaggct 300
   ctgcagtccg cttctcctgt cccctcgccc ctccacaggt ggcaagcagc acattcttgc 360
   ttacagagtc cagaaaacca cacacacaca cacacacaca cacacacaca cacacacaca 420
   aaaaaaaaca cttgccgacg agacagcccg cacttggtac ccgcctcccc atgctgcttc 480
   tcccggctct gagccgtggg tacaacccct cggggggggg ggggggagga tttctctccc 540
   ccacccccag tcttcctagc agatgtgcat cccggccaac atggagggaa atggacaacc 600
   ttgccgggga cttttttttc tttcatttga aaccatgacc gcagccgttc ctccaacctg 660
   gcctgacctc tccacgtgtc caaggaggaa gccccctggc ccagttgagg cctcgccaac 720
   ctggcacccc tccaggctcc tcctcctgct ccaaccttta aatgcatccc ggggccccag 780
   aacaccatcc gacacctgcc tgctgcccac caccagccta ccagcggccg ccatgcg    837
<210> 20
   <211> 302
   <212> DNA
   <213> Artificial Sequence
<220>
   <223> Description of Artificial Sequence: probe 343bp EPO 2ex/3ex
<400> 20
   ggaggccgag aatatcacgg tgagacccct tccccagcac attccacaga actcacgctc 60
   agggcttcag ggaactcctc ccagatccag gaacctggca cttggtttgg ggtggagttg 120
   ggaagctaga cactgccccc ctacataaga ataagtctgg tggccccaaa ccatacctgg 180
   aaactaggca aggagcaaag ccagcagatc ctacgcctgt ggccagggcc agagccttca 240
   gggacccttg actccccggg ctgtgtgcat ttcagacggg ctgtgctgaa cactgcagct 300
   tg                                                                302
<210> 21
   <211> 962
   <212> DNA
   <213> Artificial Sequence
<220>
   <223> Description of Artificial Sequence:probe 1022bp EPO lex/3ex
<400> 21
   ccgggtccct gtttgagcgg ggatttagcg ccccggctat tggccaggag gtggctgggt 60
   tcaaggaccg gcgacttgtc aaggaccccg gaagggggag gggggtgggg cagcctccac 120
   gtgccagcgg ggacttgggg gagtccttgg ggatggcaaa aacctgacct gtgaagggga 180
   cacagtttgg gggttgaggg gaagaaggtt tggggggttc tgctgtgcca gtggagagga 240
   agctgataag ctgataacct gggcgctgga gccaccactt atctgccaga ggggaagcct 300
   ctgtcacacc aggattgaag tttggccgga gaagtggatg ctggtagcct gggggtgggg 360
   tgtgcacacg gcagcaggat tgaatgaagg ccagggaggc agcacctgag tgcttgcatg 420
   gttggggaca ggaaggacga gctggggcag agacgtgggg atgaaggaag ctgtccttcc 480
   acagccaccc ttctccctcc ccgcctgact ctcagcctgg ctatctgttc tagaatgtcc 540
   tgcctggctg tggcttctcc tgtccctgct gtcgctccct ctgggcctcc cagtcctggg 600
   cgccccacca cgcctcatct gtgacagccg agtcctgcag aggtacctct tggaggccaa 660
   ggaggccgag aatatcacgg tgagacccct tccccagcac attccacaga actcacgctc 720
   agggcttcag ggaactcctc ccagatccag gaacctggca cttggtttgg ggtggagttg 780
   ggaagctaga cactgccccc ctacataaga ataagtctgg tggccccaaa ccatacctgg 840
   aaactaggca aggagcaaag ccagcagatc ctacgcctgt ggccagggcc agagccttca 900
   gggacccttg actccccggg ctgtgtgcat ttcagacggg ctgtgctgaa cactgcagct 960
   tg 962
<210> 22
   <211> 726
   <212> DNA
   <213> Artificial Sequence
<220>
   <223> Description of Artificial Sequence: primary nucleotide structure coding regions of express vector prWhEBS-BNX
<400> 22
   gcctgctgcc accaccagac taccagcggc cgccatgggg gtgcacggtg agtactcgcg 60
   ggctgggcgc tcccgcccgc ccgggtccct gtttgaccgg gatttagcgc cccggctatt 120
   ggccaggagg tggctgggtt caaggaccgg cgacttgtca aggaccccgg aagggggagg 180
   ggggtggggc agcctccacg tgccagcggg gacttggggg agtccttggg gatggcaaaa 240
   acctgacctg tgaaggggac acagtttggg ggttgagggg aagaaggttt gggggttctg 300
   ctgtgccagt ggagaggaag ctgataagct gataacctgg gcgctggagc caccacttat 360
   ctgccagagg ggaagcctct gtcacaccag gattgaagtt tggccggaga agtggatgct 420
   ggtagctggg gggtggggtg tgcacacggc agcaggattg aatgaaggcc agggaggcag 480
   cacctgagtg cttgcatggt tggggacagg aaggacgagc ttgggcagag acgtggggat 540
   gaaggaagct gtccttccac agccaccctt ctccctcccc gcctgactct cagcctggct 600
   atctgttcta gaatgtcctg cctggctgtg gcttctcctg tccctgctgt gcgctccctc 660
   tgggcctccc agtcctgggc gccccaccac gcctcatctg tgacagccga gtcctggaga 720
   ggtacc 726
<210> 23
   <211> 1146
   <212> DNA
   <213> Artificial Sequence
<220>
   <223> Description of Artificial Sequence: primary nucleotide structure coding regions of express vector prWhEBS-BNX
<400> 23
   actcacgctc agggcttcag ggaactcctc ccagatccag gaacctggca cttggtttgg 60
   ggtggagttg ggaagctaga cactgccccc ctacataaga ataagtctgg tggccccaaa 120
   ccatacctgg aaactaggga aggagcaaag ccagcagatc ctacgcctgt ggccagggcc 180
   agagccttca gggacccttg actccccggg ctgtgtgcat ttcagacggg ctgtgctgaa 240
   cactgcagct tgaatgagaa tatcactgtc ccagacacca aagttaattt ctatgcctgg 300
   aagaggatgg aggtgagttc cttttttttt tttttccttt tcttttggag aatctcattt 360
   gcgagcctga ttttggatga aagggagaat gatcgaggga aaggtaaaat ggagcagcag 420
   agatgaggct gcctgggcgc agaggctcac gtctataatc ccaggctgag atggccgaga 480
   tgggagaatt gcttgagccc tggagtttca gaccaaccta ggcagcatag tgagatcccc 540
   catctctaca aacatttaaa aaaattagtc aggtgaagtg gtgcatggtg gtagtcccag 600
   atatttggaa ggctgaggcg ggaggatcgc ttgagcccag gaatttgagg ctgcagtgag 660
   ctgtgatcac accactgcac tccagcctca gtgacagagt gaggccctgt ctcaaaaaag 720
   aaaagaaaaa agaaaaataa tgagggctgt atggaatacg ttcattattc attcactcac 780
   tcactcactc attcattcat tcattcattc aacaagtctt attgcatacc ttctgtttgc 840
   tcagcttggt gcttggggct gctgaggggc aggagggaga gggtgacatc cctcagctga 900
   ctcccagagt ccactccctg taggtcgggc agcaggccgt agaagtcggc agggcctggc 960
   cctgctgtcg gaagctgtcc tgcggggcca gggccctgtt ggtcaactct tcccagccgt 1020
   gggagcccct gcagctgcat gtggataaag ccgtcagtgg ccttcgcagc ctcaccactc 1080
   tgcttcgggc tctgggagcc caggtgagta ggagcggaca cttctgcttg ccctttctgt 1140
   aagaag                                                            1146
<210> 24
   <211> 307
   <212> DNA
   <213> Artificial Sequence
<220>
   <223> Description of Artificial Sequence: primary nucleotide structure coding regions of express vector prWhEBS-BNX
<400> 24
   ccatctcccc tccagatgcg gcctcagctg ctccactccg aacaatcact gctgacactt  60
   tccgcaaact cttccgagtc tactccaatt tcctccgggg aaagctgaag ctgtacacag 120
   gggaggcctg caggacaggg gacagatgac caggtgtgtc cacctgggca tatccaccac 180
   ctccctcacc aacattgctt gtgccacacc ctcccccgcc actcgtgaac cccgtcgagg 240
   ggctctcagc tcagcgccag cctgtcccat ggacactcca gtgccaccaa tgacatctca 300
   ggggcca                                                           307

## Description

### Field of the Invention

The invention involves the way of preparation of expression vectors used for therapeutic protein production in the mammary gland of transgenic animals. These are mainly vectors constructed via such modification of the nucleotide sequence that aims to install a restriction site in the area of translational Kozak consensus among the nucleotides -9 up to +4. Introduction of the new restriction site in this gene region enables a defined and universal fusion of the 5'end non-translated part of the regulatory gene, controlling the gene expression in the desired tissue, with the specific structural gene, coding primary amino acid sequence of the therapeutic protein.

### Background of the Invention

### Transgenic protein - erythropoietin

The inter-species gene transfer has opened a new way to isolation of the therapeutically significant human proteins from the organisms where the functional human deoxyribonucleic acid (DNA) carrying genetic information was inserted. Transcription of the transferred DNA strand carrying a human gene leads to synthesis of the foreign "messenger" ribonucleic acid (mRNA) that is translated to the original structure of the human protein. It used to be possible to gain such proteins, which find exploitation in medicine, by expensive isolation exclusively from the human material. Currently, they can be isolated from the animal products in larger quantities. Apart from the proteins of human origin with original traits, this method allows production of the new improved variants of the proteins via genetic manipulation. That means variants of such proteins with favourable therapeutic features or with minor side effects like "humanized" antibodies. These are hybrid immunoglobulin proteins with a variable part of the animal origin linked to an invariable part of the human immunoglobulin.

The human glycoprotein erythropoietin (EPO) is one of many human proteins that are produced in the mammalian tissue cultures in vitro or isolated from the animal products. It is an acid glycoprotein with a molecular weight of 34 Kda ranging in the forms: α, β, γ. They differ to each other in the form of glycosylation and in their asial forms in which the final sugar component has been removed. *(*Dordal, M.S, Wang, F.F. Goldwasser, E. (1985) The role of carbohydrate in erythropoietin action. Endocrinology, 116:2293-9*;* Goldwasser, E. Kung, C.K. Eliason, J. (1974) The role of sialic acid in erythropoietin action. J. Biol. Chem, 249:4202-6*;* Goto, M. Murakami, A. Akai, K. Kawanishi, G. Ueda, M. Chiba, H Sasaki, R. (1989) Characterization and use of monoclonal antibodies directed against human erythropoietin that recognize different antigenic determinants. Blood, 74:1415-23*;* Lukowsky, W.A. Painter, R.H. (1972) Studies on the role of sialic acid in the physical and biological properties of erythropoietin. Can. J. Biochem, 50:909-17*;* Miyake, T. Kung, C.K. Goldwasser, E. (1977) Purification of human erythropoietin. J. Biol. Chem, 252:5558-64*;* Takeuchi, M. Inoue, N. Strickland, T.W. Kubota, M. Wada, M. Schimizu, R. Hoshi, S. Kozutsumi, H. Takasaki, S. Kobata, A. (1989) Relationship between sugar chain structure and biological activity of recombinant human erythropoietin produced in Chinese hamster ovary cells. Proc. Natl. Acad. Sci. USA, 86:7819-22*;* Takeuchi, M. Takasaki, S. Shimada, M. Kobata, A. (1990) Role of sugar chains in the in vitro biological activity of human erythropoietin produced in recombinant Chinese hamster ovary cells. J. Biol. Chem, 265:12127-30*;* Tsuda, E. Kawanishi, G. Ueda, M. Masuda, S. Sasaki, R. (1990) The role of carbohydrate in recombinant human erythropoietin. Eur. J. Biochem, 188:405-11*).*

Erythropoietin is able to stimulate the transformation of primitive embryonic cells in bone marrow to proerythroblasts that gradually mature, synthesize hemoglobin and are released to the blood circulation as erythrocytes. *(*Erslev, A. (1953) Humoral regulation of red cell production. Blood, 8: 349-357*).* Erythropoietin is found in very low concentrations in plasma. Yet this normal low concentration is sufficient to stimulate a replacement of the erythrocytes prone to hemolysis. *(*Fried, W. (1972) The liver as a source of extrarenal erythropoietin production. Blood, 40:671-7*;* Jacobson, L.O. Goldwasser, E. Plzak, L.F. Fried, W. (1957) Studies on erythropoiesis Part IV. Reticulocyte response of hypophysectomized and polycythemic rodents to erythropoietin. Proc. Soc. Exp. Biol. Med, 94:243:249*;* Zanjani, E.D. Ascensao, J.L. McGlave, P.B. Banisadre, M. Ash, R.C. (1981) Studies on the liver to kidney switch of erythropoietin production. J. Clin. Invest, 67:1183-1188*).* The amount of erythropoietin increases with the oxygen deficiency in case of reduced oxygen transfer through erythrocytes as a response to stress of the hypoxic tissue *(*Beru, N. McDonald, J. Lacombe, C. Goldwasser, E. (1986) Expression of the erythropoietin gene. Mol. Cell. Biol, 6:2571-5*;* Bondurant, M.C. Koury, M.J. (1986) Anemia induces accumulation of erythropoietin mRNA in the kidney and liver. Mol. Cell. Biol, 6:2731-3*;* Lacombe, C. Da Silva, J.L. Bruneval, P. Fournier, J.G. Wendling, F. Casadevall, N.Camilleri, J.P. Bariety, J.Varet, B. Tambourin, P (1988) Peritubular cells are the site of erythropoietin synthesis inthe murine hypoxic kidney. Clin. Invest, 81:620-623*;* Yanjani, E.D. Peterson, E.N. Gordon, A.S. Waterman, L.R. (1974) Erythropoietin production in the fetus: role of kidney and maternal anemia J. Lab. Clin. Med, 83:281-7*; Shuster SJ, et al. 1987*)*.* Due to its effects is erythropoietin suitable for diagnostics and therapy of the blood disorders characterized by a defective formation of erythrocytes. The human erythropoietin nucleotide sequence was gained and published by authors *(*Jacobs, K. Shoemaker,C. Rudersdorf, R. Neill, S.D. Kaufman, R.J. Mufson, A. Seehra, J. (1985) Isolation and characterization of genomic and cDNA clones of human erythropoietin. Nature, 313:806-10*;* Lin, F.K. Suggs, S. Lin, C.H. Browne, J.K. Smalling, R. Egrie, J.C. Chen, K.K. Fox, G.M. Martin, F. Stabinsky, Z. (1985) Cloning and expression of the human erythropoietin gene. Proc. Natl. Acad. Sci. USA, 82:7580-4*).* Biologically active erythropoietin was initially prepared on stably transfected liver cells of African green monkey *(*Jacobs, K. Shoemaker, C. Rudersdorf, R. Neill, S. D. Kaufman, R.J. Mufson, A. Seehra, J. (1985) Isolation and characterization of genomic and cDNA clones of human erythropoietin. Nature, 313:806-10*)* and Chinese hamster ovary cells of (CHO) *(*Lin, F.K. Suggs, S. Lin, C.H.Browne, J.K.Smalling, R.Egrie, J.C. Chen, K.K. Fox, G.M. Martin, F. Stabinsky, Z. (1985) Cloning and expression of the human erythropoietin gene. Proc. Natl. Acad. Sci. USA, 82: 7580-4*).* Later it was also isolated from other mammalian systems like transformed human foetal liver cells *(*Browne, J.K. Cohen, A.M. Egrie, J.C. Lai, P.H. Lin, F.K. Strickland, T. Watson, E. Stebbing, N. (1986) Erythropoietin: gene cloning, protein structure, and biological properties. Cold. Spring Harb. Symp. Quant. Biol, 1:693-702*),* in B-lymphoblastoid cells *(*Yanagi, H. Yoshima, T. Ogawa, I. Okamoto, M. (1989) Recombinant human erythropoietin DNA produced by Namalwa cells. DNA, 8:419-27), from melanoma cells *(*Keay, L. Walsh, J.M. Buchholz, R.M. Terando, J.M. (1990) Production of recombinant human erythropoietin in Bowes melanoma cells in suspension culture. Appl. Microbiol. Biotechnol, 34:198-202*)* and hamster liver cells *(*Brody VC et al. 1988; Dube, S. Fisher, J.W. Powell, J.S. (1988) Glycosylation at specific sites or erythropoietin is essential for biosynthesis, secretion, and biological function. J. Biol. Chem, 263:17516-21*;* Powell, J.S. Berkner, K.L. Lebo, R. V. Adamson, J.W. (1986) Human erythropoietin gene: high level expression in stably transfected mammalian cells and chromosome localization. Proc. Natl. Acad. Sci. USA, 83:6465-9*;* Tsuda, E. Goto, M. Murakami, A. Akai, K. Ueda, M. Kawanishin, G. Takahashi, N. Sasaki, R. Chiba, H. Ishihara, H. (1988) Comparative structural study of N-linked oligosaccharides of urinary and recombinant erythropoietins. Biochemistry, 27:5646-54*).* When the gene for erythropoietin is inserted into cells of invertebrates or prokaryote organisms as well as to insect cells *(*Quelle, F.W. Caslake, L.F. Burkert, R.E. Wojchowski, D.M. (1989) Hihg-level expression and purification of a recombinant human erythropoietin produced using a baculovirus vector. Blood, 74:652-7*)* to bacteria's *(*Lee-Huang, S. (1984) Cloning and expression of human erythropoietin cDNA in Escherichia coli. Proc. Natl. Acad. Sci. USA, 81:2708-12*)* or to yeast *(*Elliott, S.Giffin, J. Suggs, S. Lau, E.P. Banks, A.R. (1989) Secretion of glycosylated human erythropoietin from yeast directed by the alpha-factor leader region. Gene, 79:167-80*),* it lacks sugar structures that are in native condition present on human hormone isolated from the mammalian transfected cells. Most studies utilize transfected Chinese hamster ovary cells for the expression of erythropoietin gene (CHO) *(*Browne, J.K. Cohen, A.M. Egrie, J.C. Lai, P.H. Lin, F.K. Strickland, T. Watson, E. Stebbing, N. (1986) Erythropoietin: gene cloning, protein structure, and biological properties. Cold. Spring. Harb. Symp. Quant. Biol, 1:693-702*;* Davis, J.M. Arakawa, T. Strickland, T.W. Yphantis, D.A. (1987) Characterization of recombinant human erythropoietin produced in Chinese hamster ovary cells. Biochemistry, 26:2633-8*;* Delorme, E. Lorenzini, T. Giffin, J. Martin, F. Jacobsen, F. Boone, T. Elliott, S. (1992) Role of glycosylation on the secretion and biological activity of erythropoietin. Biochemistry, 31:9871-6*;* Recny, M.A. Scoble, H.A. Kim, Y. (1987) Structural characterization of natural human urinary and recombinant DNA-derived erythropoietin. Identification of des-arginine 166 erythropoietin. J. Biol. Chem, 262:17156-63*;* Sasaki, H. Bopthner, B. Dell, A. Fukada, M. (1987) Carbohydrate structure of erythropoietin expressed in Chinese hamster ovary cells by a human erythropoietin cDNA J. Biol. Chem, 262:12059-76*;* Sasaki, H. Ochi, N. Dell, A. Fukuda, M. (1988) Site-specific glycosylation of human recombinant erythropoietin: analysis of glycopeptides or peptides at each glycosylation site by fast atom bombardment mass spectrometry. Biochemistry, 27:8618-26*;* Takeuchi, M. Inoue, N. Strickland, T.W. Kubota, M. Wada, M. Shimizu, R. Hoshi, S. Kozutsumi, H. Takasaki, S. Kobata, A. (1989) Relationship between sugar chain structure and biological activity of recombinant human erythropoietin produced in Chinese hamster ovary cells. Proc. Natl. Acad. Sci. USA, 86:7819-22*).* The expressed recombinant erythropoietin is likewise glycosyled in the CHO cells as in human cells. Hence, the CHO cells are utilized for erythropoietin production applicable in pharmacy *(*Browne, J.K. Cohen, A.M. Egrie, J.C. Lai, P.H. Lin, F.K. Strickland, T. Watson, E. Stebbing, N. (1986) Erythropoietin: gene cloning, protein structure and biological properties. Cold. Spring. Harb. Symp. Quant. Biol, 1:693-702*;* Egrie, J.C. Browne, J. Lai, P. Lin, F.K. (1985) Characterization of recombinant monkey and human erythropoietin Prog. Clin. Biol. Res, 191:339-50*).* The main disadvantage of the current way of erythropoietin preparation consists in generally high costs and demanding tissue culture labour that reflects on high final price of the recombinant erythropoietin. Considering the production height requirements, erythropoietin, produced in the mouse and rabbit mammary gland respectively, is a suitable model protein for verification of our suggested strategy of preparation and testing of the function gene constructs. As the stimulation of transformation of the primitive embryonic cells in bone marrow to proerythroblasts does not require relatively high doses of human erythropoietin, transgenic rabbit represents in this case, and many other cases of similar effective stimulation factors, not only an optimal testing, but also a target production organism.

### DNA transfer

Microinjection of genetically manipulated DNA into the male pronucleus of the fertilized oocyte is the most frequently used method for transfer of the heterogenous DNA into mammalian cells and following production of transgenic animals *(*Brinster, R.L. Chen, H. Y. Trumbauer, M.E. Avarbock, M.R. (1980) Translation of globin messenger RNA by the mouse ovum. Nature, 283:499-501*;* Brehm, G. Brenic, H.M. Goodman, R. C. Selden, F. Kraublish, H. (1985) Production of transgenic mice, rabbits and pigs by microinjection into pronuclei Zuchthygiene, 20:251-252*;* Constantini, F. Lac, E. (1982) Gene transfer into the mouse germ-line. J. Cell. Physiol. Suppl, 1:219-26*;* Gordon, J.W. Ruddle, F.H. (1981) Integration and stable germ line transmission of genes injected into mouse pronuclei. Science, 214:1244-6*;* Hogan, B. Constantini, Lacy E (1986). Manipulating the mouse embryo. Laboratory manual. N. Y. Cold Spring Harbor Laboratory*),* or such approach is used for transfer, where initially, the fertilized mammalian oocyte is deprived of nucleus (oocyte enucleation) and subsequently another cell nucleus is introduced by transfer from normal or genetically manipulated mammalian cells; these are mainly pluripotent embryonic stem cells (ES) or terminally differentiated but re-programmable cells of fibroblastoid origin (nuclear transplantation method, a part of so-called cloning) *(*McGrath, J. Solter, C. (1983) Nuclear transplantation in the mouse embryo by miscrosurgery and cell fusion. Science, 220:1300-2*;* McGrath, J. Solter, D. (1983) Nuclear transplantation in mouse embryos. J. Exp. Zool, 228:355-62*;* McGrath, J. Solter, D. (1984) Maternal Thp lethality in the mouse is a nuclear, not cytoplasmic, defect. Nature, 308:550-1*;* McGrath, J. Solter, D. (1984) Completion of mouse embryogenesis requires both the maternal and paternal genomes. Cell, 37:179-83*;* Surani, M.A. Barton, S.C. (1983) Development of gynogenetic eggs in the mouse: implications for parthenogenetic embryos. Science, 222:1034-6*;* Surani, M.A. Barton, S.C. Norris, M.L. (1984) Development of reconstituted mouse eggs suggests imprinting of the genome during gametogenesis. Nature, 308:548-50*).* In both methods, the exogenous DNA is transferred into the cell nucleus, where it is randomly integrated in the host genome by nuclear reparative mechanisms. Using this way it is not possible to control either the integration site in the host genome or number of the integrated specific gene copies. Benefit of the method utilizing nuclear transplantation is the possibility to identify and characterize a suitable clone for development of a transgenic animal already during in vitro cultivation of the transfected cells. It reduces the time delay caused by the essential generation time needed to search out the positive transgenic individuals commonly gained by microinjection technique. It leads to the more effective transgenic animal development, as the extent testing of many natal individuals is not required.

A better alternative of inserting exogenous DNA in the host genome is the utilization of target genome fragment insertion using homologous recombination between gene locus of the mammalian embryonic stem cell (or mammalian fibroblast) and heterogenous recombinant DNA in the exogenous vector form. Recombination induced by the nuclear enzymes leads to the target mutation into the genome along with a control of the integration site and of the number of integrated gene copies. *(*Doetschman, T. Gregg, R.G. Maeda, N. Hooper, M.L. Melton, D.W. Thompson, S. Smithies, O. (1987) Targetted correction of a mutant HPRT gene in mouse embryonic stem cells Nature, 330:576-8*;* Mansour, S.L. (1990) Gene targeting in murine embryonic stem cells: introduction of specific alterations into the mammalian genome. Genet. Anal. Tech. Appl. 7:219-27*;* Robertson, E.J. (1991) Using embryonic stem cells to introduce mutations into the mouse germ line. Biol. Reprod, 44:238-45*).* This approach enables the transgene targeting or controlled insertion of the desired mutation for the loss of the endogene function, so-called "gene knock-out".

### Construction of the expression vectors for protein production in the mammary gland

The key step for development of genetically manipulated animals is a construction of the expression vector derived from the bacterial plasmid DNA, to which the required mammalian gene, specified for production of therapeutic protein, will be inserted. To stimulate genomic expression, other DNA sequences derived from the mammalian genes specifically expressed in desired tissue are fused with to the coding sequences of the manipulated gene. The resulting expression vector is a complex DNA molecule where single parts of different origin link in one functional unit. The way of structure gene expression depends on the mutual interactions with the integration site in the recipient genome and on the functionality of the fused regulatory sequences jointly transferred.

The mammalian mammary gland is suitable organ for the heterogenous transgenic protein production with the therapeutic utilization in human medicine. For this reason the interest is focused on the study of regulatory gene regions that code for the important milk proteins such as β-lactoglobulin, α-lactalbumin, β-casein and the whey acid protein (WAP). Regulatory sequences of these genes are used to construct expression vectors and to regulate heterogenous gene production in the animal mammary gland. For development of a transgenic animal, there is usually used a gene whose product is the main protein component in the specific animal species milk and currently is produced specifically in the animal organism in the mammary gland. *(*Devinoy, E. Hubert, C. Jolivet, G. Thepot, D. Clerque, N. Desaleux, M. Dion, M. Servely, J.L. Houdebine, L.M. (1988) Recent data on structure of rabbit milk protein genes and on the mechanism of the hormonal control of their expression. Reprod. Nutr. Develop, 28:1145-64*;* Hennighausen, L.G. Sippel, A.E. (1982) Characterization and cloning of the mRNAs specific for the lactating mouse mammary gland. Eur. J. Biochem, 125:131-41*;* Hobbs, A.A. Richards, D.A. Kessler, D.J. Rosen, J.M. (1982) Complex hormonal regulation of rat casein gene expression. J. Biol. Chem. 257:3598-605*).* The type of target production animal usually regulates choice of the milk protein gene regulation region that will be used to construct the expression vector. The dominant protein produced specifically in the mouse, rabbit and other rodent mammary gland is WAP *(*Campbell, S.M. Rosen, J.M. Henninghausen, L.G. Strech-Jurk, U. Sippel, A.E. (1984) Comparison of the whey acidic protein genes of the rat and mouse. Nucleic. Acids. Res, 12:8685:97*.* Grabowski, H. LeBars, D. Chene, N. Attal, J. Maliénou-N'Gassa, R. Puissant, D. Houdebine, L.M. (1991) Rabbit whey acidic protein concentration in milk, serum, mammary gland extract and culture medium. J. Dairy Sci. 74:4143-54*;* Hennighausen, L.G. Sippel, A.E. Hobbs, A.A. Rosen, J.M. (1982) Comparative sequence analysis of the mRNAs coding for mouse and rat whey protein. Nucleis Acids Res. 10:3733-44*);* Piletz, J.E. Heinlen, M. Ganschow, R.E. (1981) Biochemical characterization of a novel whey protein from murine milk. Biol. Chem, 256:11509-16*).* Regulatory sequences of this gene are therefore suitable for preparation of expression vectors regulating the protein production in mammary gland of these animals *(*Kolb, A.F. Gunzburg, W.H. Albang, R. Brem, G. Erfle, V. Salmons, B. (1994) Negative regulatory element in the mammary specific whey acidic protein promoter. Cell. Biochem. 56:245-61*).* At present time, a whole range of genes has been cloned to various expression vectors with milk protein promoter sequences including vectors using the regulatory sequences from WAP gene. *(*Andres, A.C. Shoenenberger, C.A. Groner, B. Hennighausen, L. LeMeur, M. Gerlinger, P. (1987) Ha -ras oncogene gene expression directed by a milk protein gene promoter: tissue specifity, hormonal regulation and tumor induction in transgenic mice. Proc. Natl. Acad. Sci. USA, 84:1299-1303*;* Devinoy, E. Thépot, D. Stinnakre, M.G. Fontaine, M.L. Grabowski, H. Puissant, C. Pavirani, A. Houdebine, L.M. (1994) High level production of human growth hormone in the milk of transgenic mice: the upstream expression to the mammary gland. Transgenic. Res, 3:79-89*;* Gordon, K. Lee, E. Vitale, J.A. SMith, A.E. Westphal, H. Hennighausen, L. (1992) Production of human tissue plasminogen activator in transgenic mouse milk Biotechnology, 24:425-8*;* Gunzburg, W.H. Salmons, B. Zimmermann, B. Muller, M. Erfle, V. Brem, G. (1991) A mammary specific promoter directs expression of growth hormone not only to the mammary gland, but also to Bergman glia cells in transgenic mice. Mol. Endocrinol, 5:123-33*;* Limonta, J. Pedraza, A. Rodriquez, A. Freyre, F.M. Barral, A.M. Castro, F.O. Lleonart, R. Gracia, C.A. Gavilondon, J.V. de la Fuente, J. (1995) Production of active anti-CD6 mouse/human chimeric antibodies in the milk transgenic mice. Immunotechnology, 1:107-13*;* Limonta, J.M. Castro, F.O. Martinez, R. Puentes, P. Ramos, B. Aguilar, A. Lleonart, R.L. de la Fuente, J. (1995) Transgenic rabbits as bioreactors for the production of human growth hormone. J. Biotechnol, 40:49-58*;* Pittius C. W. Heninghausen, L. Lee, E. Westphal, H. Nichols, E. Vitale, J. Gordon, K. (1988) A milk protein gene promoter directs the expression of human tissue plasminogen activator cDNA to the mammary gland in transgenic mice. Proc. Natl. Acad. Sci. USA, 85:5874-8*;* Reddy, V.B. Vitale, J.A. Wei, C. Montaya-Zavala, M. Stice, S.L. Balise, J. Robl, J.M. (1991) Expression of Human Growth Hormone in the milk of transgenic mice. Animal Biotechnology, 2:15-29*;* Thepot. D. Devinoy, E. Fontaine, M.L. Stinnakre, M.G. Massound, M. Kann, G. Houdebine, L.M. (1995) Rabbit whey acidic protein gene upstream region controls high-level expression of bovine growth hormone in the mammary gland of transgenic mice. Mol. Reprod. Dev, 42:261-7*;* Tomasetto, C. Wolf, C. Rio, M.C. Mehtali, M. LeMeur, M. Gerlinger, P. Chambon, P. Lathe, R. (1995) Brest Cancer Protein PS2 Synthesis in Mammary Gland of Transgenic Mice and secretion into Milk. Mol Endocrinol, 89:1579-84*;* Velander, W.H. Page, R.L. Morcol, T. Russell, C.B. Canseco, R. Young, J.M. Drohan, W.N. Gwazdauskas, F.C.Wilkins, T.D. Johnson, J.L. (1992) Expression of human protein C in the milk of transgenic mice and pigs. Ann. N. Y. Acad. Sci, 665:391-403*;* Yu, S.H. Deen, K.C. Lee, E. Hennighausen, L. Sweet, R.W. Rosenberg, M. Westphal, H. (1989) Functional human CD4 protein produced in milk of transgenic mice. Mol. Biol. Med, 6:255-61*).* The main significant sequence motifs participating in process of the transcription regulation were localized in region within the distance of 6.3 kb from the start codon ATG *(*Devinoy, E. Malienou - N'Gassa, R. Thepot, D. Puissant, C. Houdebine, L.M. (1991) Hormone responsive elements within the upstream sequences of the rabbit whey acidic protein (WAP) gene direct chloramphenicol acetyl transferase (CAT) reporter gene expression in transfected rabbit mammary cells Mol. Cell. Endocrinol. 81:185-93*)* The vectors for expression of the hEPO structure gene were also constructed in a similar way, using the same regulatory regions. *(*Aquirre, A. Castro-Palomino, N. De la Fuente, J. Castro, F.O. (1998) Expression of human erythropoietin and of the endogenous WAP gene in th emammary gland of transgenic rabbits during gestation and lactation. Transgenic. Res, 4:311-7*;* Massoud, M. Attal, J. Thepot, D. Pointu, H. Stinnakre, M.G. Theron, M.C. Lopez, C. Houdebine, L.M. (1996) The deleterious effects of human erythropoietin gene driven by the rabbits whey acidic protein gene promoter in transgenic rabbits. Reprod. Nutr. Dev, 36:555-63*;* Rodriguez, A. Castro, F.O. Aguilar, A.Ramos, B. Del Barco, D.G. Lleonart, R. De la Fuente, J. (1995) Expression of active human erythropoietin in mammary gland of lactating transgenic mice and rabbits. Biol. Res, 28:141-53*).* It proves that in many cases, the regulatory regions function as well and sometimes even better in the organism of animal species of different origin than the one where milk protein gene was derived. The reason is mainly the fact that the number of integrated copies and the chromatin transcriptional activity in the integration region play an important role concerning quantity and protein production specificity. An important role is also played by other regulatory sequences in introns and in 3'terminal region *(*Dale, T.C. Krnacik, M.J. Schmidhauser, C. Yang, C.L-Q. Bissell, M.J. Rosen, J.M. (1992) High-level expression of the rat Whey acidic protein gene is mediated by elements in the promoter and 3'untranslated region. Mol. And Cel. Biol. 12:905-914*)* and also in bigger distance upstream from ATG *(*Bischoff, R. Degryse, E. Perraund, F.Dalemans, W. Ali-Hadji, D. Thepot, D. Devinoy, E. Houdebine, L.M. Pavirani, A. (1992) A 17.6 kbp region located upstream of the rabbit WAP gene directs high level expression of a functional human protein variant in mose milk. FEBS Lett, 6:265-8*).* These regulatory sequences are able to influence significantly as expression level so to modify its tissue specificity. However, introns influence gene expression even if they don't harbour regulatory sequences; since the expression vectors formed by genome sequences of the genes with introns usually have higher expression levels than vectors constructed on the basis of genes transcribed via reverse transcription from mRNA (cDNA) *(*Brinster, R.L. Allen, J.M. Behringer, R.R. Gelinas, R.E. Palmiter, R.D. (1988) Introns increase transcriptional efficiency in transgenic mice. Proc. Natl. Acad. Sci. USA, 85:836-40*;* Choi, T. Huang, M. Gorman, C. Jaenisch, R. (1991) A generic intron increases gene expression in transgenic mice. Mol.Cell.Biol, 11:3070-4*;* Palmiter, R.D. Sandgren, E.P. Avarbock, M.R. Allen, D.D. Brinster, R.L. (1991) Heterologous introns can enhance expression of transgenes in mice. Proc. Natl. Acad. Sci. USA, 88: 478-82*).* On the other hand, the introns derived from hEPO gene can contain regulatory elements with negative regulatory efficiency (silencers) that could silence the gene transcriptional activity. The results of various authors bring a range of findings that we can't unambiguously conclude at present. It appears that the promoter region up to 6.3-kb upstream from ATG does not harbour the main regulatory elements that are able to ensure high and tissue specific expression along with the regulatory intron sequences and with the 3'nontranslated WAP gene region. However, if the WAP gene structure region is replaced by the structural genome segment, or by (cDNA) of other gene, it usually reduces the expression depending largely on the chromatin transcriptional activity round the integration site and on the number of transgene copies in the genome. The tissue specificity is often modified too; it leads to an undesirable (ectopic) expression of the transferred gene also in other tissues outside the mammary gland. So a heterogenous protein can negatively influence the animal health. *(*Massoud, M. Attal, J. Thepot, D. Pointu, H. Stinnakre, M.G. Theron, M.C. Lopez, C. Houdebine, L.M. (1996) The deleterious effects of human erythropoietin gene driven by the rabbits whey acidic protein gene promoter in transgenic rabbits. Reprod. Nutr. Dev, 36: 555-63*).* The basic requirement on the expression vectors that are used for gene manipulation at the evolutionary higher organisms, is apart from the desired protein production height target expression of the gene specified for the desired protein production into an existing tissue of the host animal. A common feature of the vectors that utilize promoter sequences of rabbit WAP gene for regulation of the human erythropoietin gene expression is cloning of the structural erythropoietin gene in the form of genome DNA or cDNA sequences into the region among coding sequences of structural WAP gene. It is advantageous that the regulatory function of the 5'terminal region of WAP gene isn't dirupted and the introns of the driving gene can participate on the gene expression control. The human erythropoietin expression profile should conform the natural WAP gene expression. Production of the fusion protein at the gene fusion is a considerable disadvantage of the majority of these vectors. It is not possible to estimate in advance how the amino acids of the WAP region influence human erythropoietin activity and how the technological approaches intent on their elimination would influence the erythropoietin activity. It is possible to prevent these complications according to the invention for construction of hybrid vectors for the preparation of transgenic animals. That consists in the utilization of the inserted restriction motif for *NotI* and *NcoI* sites in the Kozak consensus sequence. For these reasons these vectors are further developed and simultaneously other sequence motifs are explored and attached. The possibilities of connection of gene segments are also explored. They can influence the transcriptional and translational activity of the DNA inserted in the host genome and its translation to RNA. Even authors of the invention suggested this.

### Kozak consensus for the translational initiation region

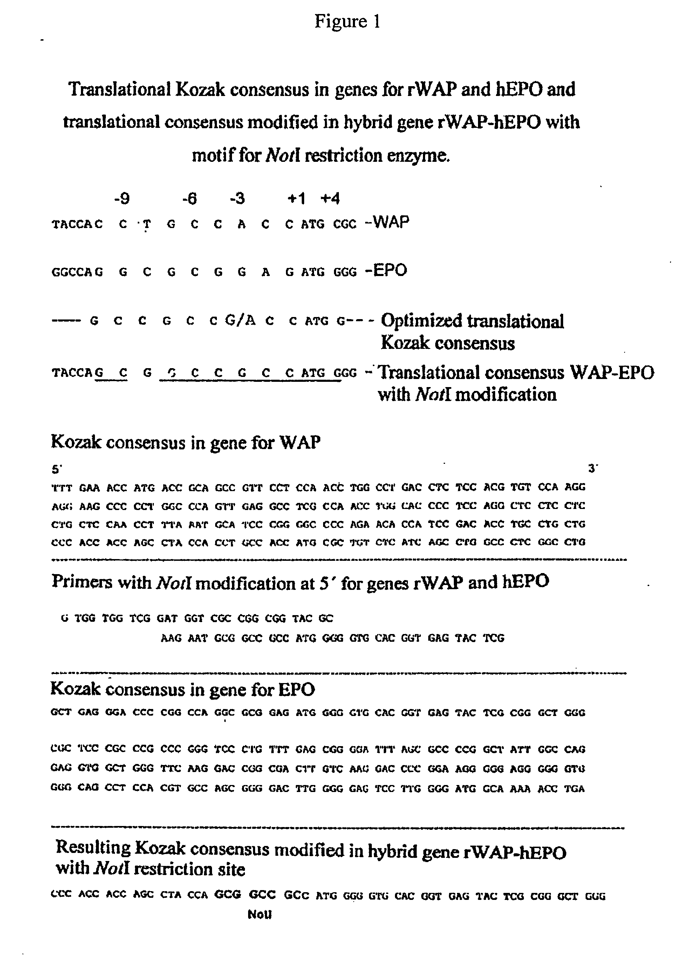

Model for the mechanism of translational regulation in the eukaryots, called "scanning mechanism for initiation of translation", was suggested by Marylin Kozak more than 20 years ago *(*Kozak, M Shatkin, A.J. (1978) Identification of features in 5'terminal fragments from retrovirus mRNA which are important for ribosome binding. Cell, 13:201-12*;* Kozak, M. Shatkin, A.J. (1979) Characterization of translational initiation regions from eukaryotic messenger RNAs. Methods. Enzymol, 60:360-75*).* This model assumes that a small subunit of the eukaryotic ribosome (40S) that carries RNA with a bound methionine (Met-RNA), first binds to 5' by the methylation of modified messenger RNA, then it migrates along the RNA molecule up to the start "favoured" AUG codon (triplet adenine - uracil - thymine) where the migration terminates and the process of translation begins. This model postulates the necessity of two basic factors for the choice of the favoured first AUG namely a position closed to 5' RNA then the context, thus information content or the sequences closed to the start AUG. Proof of "the rule for the first AUG" was demonstrated by Kozak in 1987. She proved by analysis of the sequences round the start AUG in 699 various vertebrate RNA that "the rule for the first AUG" applies to 90-95% of the tested RNA types. For the first time she introduced a nucleotide sequence GCCGCCA/GCCAUGG as the sequence consensus for the translation initiation in higher eukaryots *(*Kozak, M. (1987) At least six nucleotides preceding the AUG initiator codon enhance translation in mammalian cells. J. MoL Biol., 196:947-50*)* (see Figure 1). In other experiments with the use of locally-redirected mutagenesis on the model of preproinsulin gene inserted to apish CV-1 renal cells that are transformed by a mutant virus SV-40 (COS) cells, Kozak demonstrated the importance of the positions G+4 and other nucleotides in the sequence consensus at positions -1 to -6 *(*Kozak, M. (1986) Bifunctional messenger RNAs in eukaryotes. Cell, 47:481-3*;* Kozak, M. (1986) Influences of mRNA secondary structure on initiation by eukaryotic ribosomes. Proc. Natl. Acad. Sci. USA, 83:2850-4*;* Kozak, M. (1987) At least six nucleotides preceding the AUG initiator codon enhance translation in mammalian cells. J. Mol. Biol, 196:947-50*;* Kozak, M. (1980) Influence of mRNA secondary structure on binding and migration of 40S ribosomal subunits. Cell, 19: 79-90*).* The importance of purine at position -3 was besides the target mutagenesis confirmed by the discovery of Thalassaemia type at which the modification in sequence from CACCAUG to CCCCAUG drastically inhibits translation initiation of α-globin. *(*Morlé, F. Lopez, B. Henni, T. Godet, J. (1985) alpha-Thalassaemia associated with the deletion of two nucleotides at position -2 and -3 preceding the AUG codon. EMBO J, 4:1245-50*)*. Purine at position -3 (usually A) is the most conservative nucleotide in all eukaryotic RNAs including vertebrate, in fungi *(*Paluh, J.L. Orbach, M.J.Legerton, T.L. Yanofsky, C. (1988) The cross-pathway control gene of Neurospora crassa, cpc-1, encodes a protein similar toGCN4 of yeast and the DNA-binding domain of the oncogene ν-jun-encoded protein. Proc. Natl. Acad. Sci. USA, 85:3728-32*)* and in plants *(*Heidecker, G. Messing, J. (1987) A method for cloning full-length cDNA in plasmid vectors. Methods. Enzymol, 154:28-41*).* The directed mutation at position -3 influences translation more than the site mutation of any other nucleotide in the sequence consensus *(*Kozak, M. (1986) Influences of mRNA secondaaary structure on initiation by eukaryotic ribosomes. Proc. Natl. Acad. Sci. USA, 83:2850-4*).* Other studies discovered that in the presence of adenine at position -3, the positions of other nucleotides have only a side effect on the initiation translation efficiency. However, in case of purine absence at position -3 the presence of guanine G+4 is critical for the efficient translation and the function of nucleotides in other positions enhances, especially at position -6 and -9 *(*Kozak, M. (1987) At least six nucleotides preceding the AUG initiator codon enhance translation in mammalian cells. J. Mol. Biol, 196: 947-50*).* For practical reasons the author suggested to distinguish a strong and a weak initiation codon according to positions -3 and +4 *(*Kozak, M. (1989) Context effects and inefficient initiation at non-AUG codons in eukaryotic cell-free translation systems. Mol. Cell. Biol, 9:5073-80*;* Kozak, M. (1989) Circumstances and mechanisms of inhibition of translation by secondary structure in eukaryotic mRNAs. Mol. Cell. Biol, 9:5134-42*).* For this reasons it is useful to prepare expression vector by connecting 5'regulatory sequences through the structural gene in the region of the start codon adenine-thymine-guanine (ATG) as the authors of this invention managed.

### Summary of the invention

Some of the stated disadvantages of the existing situation, involving methods of the expression vector construction and methods of recombinant erythropoietin preparation, are eliminated by the way of preparation of expression vectors for therapeutic protein production in transgenic animals based on this invention. Its principle consists in using the sequence motif for restriction endonucleases *Not*I or *NcoI* for integration of gene segment involving regulatory promoter region with the gene segment involving structural fragments of gene coding the desired protein. The sequence motif is composed by the modification of original Kozak consensus in the region among nucleotides -9 to +4 resulting in the hybrid recombinant expression vector for therapeutic protein production.

Further the subject matter of the invention consists in modification of translation Kozak consensus of genes for rabbit acid WAP protein and for human erythropoietin by the insertion of sequence motif for a restriction endonuclease namely *Not*I in the region among nucleotides -9 to +4 in order to fuse the selected gene segments. It results in the expression vector prWhEBS-BNX for human erythropoietin production in mammary gland of the transgenic organisms.

Further the subject matter of the invention consists in a primary nucleotide DNA structure of the vector prWhEBS-BNX prepared according to the invention and other vectors that derived from the original vector prWhEBS-BNX via a further modification.

Likewise the eukaryotic cells, carrying in their genome a nucleotide primary structure of the hybrid genes derived from expression vectors prepared according to the invention, are the subject matter of the invention.

The subject matter of the invention is also non-human transgenic organisms carrying in their genome a nucleotide primary structure of the hybrid genes derived from expression vectors prepared according to the invention.

The preparation of expression vectors according to the invention makes use of the fact that the motif coding amino acid methionine is a universal triplet for all eukaryotic genes and so-called optimized sequence consensus for the most effective translational efficiency is known for the Kozak sequence motif. These facts gave base to an idea of a possibility to prepare the universal applicable sequence close to the optimal variant with artificially installed restriction sites suitable for cloning expression vectors. In such expression vectors it would be possible to connect through the promoter regulatory sequences directly with the first ATG triplet of the specific gene.

In reference to the application of other restriction sites for cloning longer fragments, only the ones that rarely occur in the host genome DNA are structurally interesting. One of such enzymes is *NcoI* that distinguishes the 6-mer-restriction site CCATGG, located also in the optimized variant of the Kozak consensus. Another, even more useful cloning-wise is the restriction site *NotI* (GCGGCCGC, see Figure 1) that rarely occurs in a mammalian genome, say with the average occurrence frequency 1:10⁶ base pairs. After inserting of this restriction motif to the Kozak consensus, the modification of the optimized consensus will be minimal and it will not reduce the translational efficiency at the product synthesis.

The method according to the invention imaginatively utilizes Kozak sequence motif to a target modification of primary nucleotide DNA sequence leading to the unique installation of the restriction site for *NotI* or *NcoI*, was not used yet; it faces to the preparation of a universal vector regulating protein production in the mammalian mammary gland with a significantly simplified possibility to replace the structural gene for production of the specific protein. Thus the method according to the invention enables faster preparation of expression vector for a production of a range of therapeutic proteins; it will result in a wide use and in shortened time required to develop expression vectors.

### Description of the Drawings

- Figure 1:: Primary nucleotide structure of translational Kozak consensus genes for rWAP and hEPO; their connection through the translational Kozak consensus *NotI* modification.
- Figure 2:: The preparation of probes suitable for identification of phage clones carrying a segment of primary nucleotide structure of gene for rWAP. The probes were prepared by PCR reaction using primers (underlined segments of primary sequence).

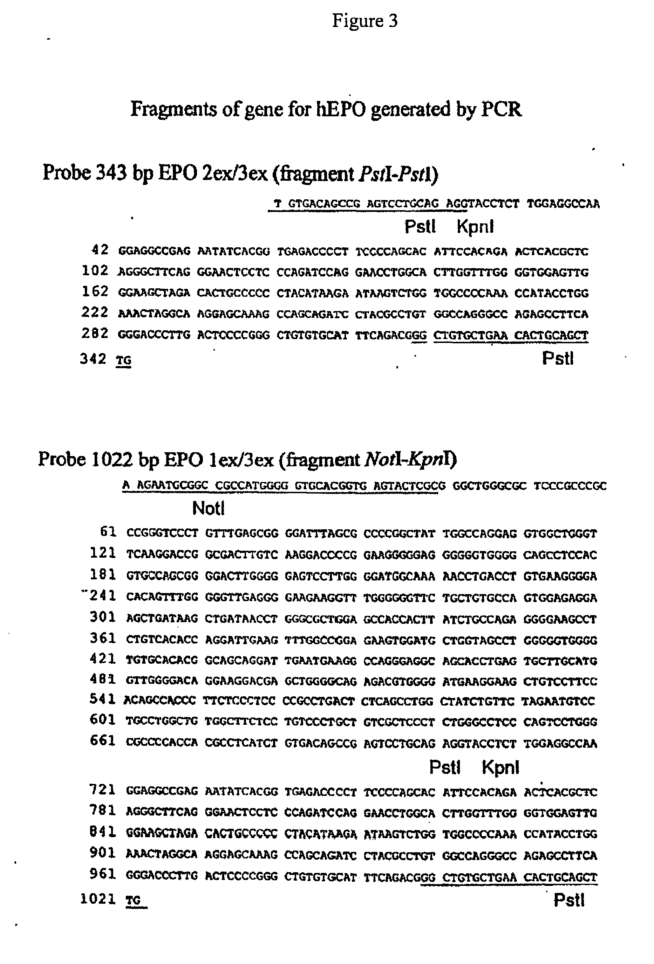

- Figure 3:: Preparation of probes suitable for identification of phage clones carrying a segment of primary nucleotide structure of gene for hEPO. The probes were prepared by PCR reaction using primers (underlined segments of primary sequence).
- Figure 4:: Electrophoretic representation of the amplification of probes used to search for phage clones carrying primary nucleotide structure of genes for rWAP and hEPO.
- Figure 5:: Ability verification of amplified probes to hybridize under the stringent conditions with genome DNA modified by restriction enzymes and bound on a nylon membrane by "Southern blot".
- Figure 6:: Restriction maps of isolated phage clones carrying primary nucleotide structures of genes for rWAP and for hEPO. The gene structure segments representing introns and exons (wide segments are exons, thinner segments are introns) and most important restriction sites are denoted.
- Figures 7-10:: Simplified diagrams depicting the procedure of restriction digestion of fragments derived from isolated phage clones rWAP and hEPO and vector pBS. Final expression vector prWhEBS-BNX construction through the modification of translational Kozak consensus with inserted *NotI* restriction site.
- Figure 11:: Primary nucleotide structure coding regions for expression vector prWhEBS-BNX.

### Examples

The following examples serve for better understanding of the invention without limitations.

### Example 1: Sub-cloning of 5' region rWAP gene from DASH II phage genome library to plasmid prW5'BS-KE

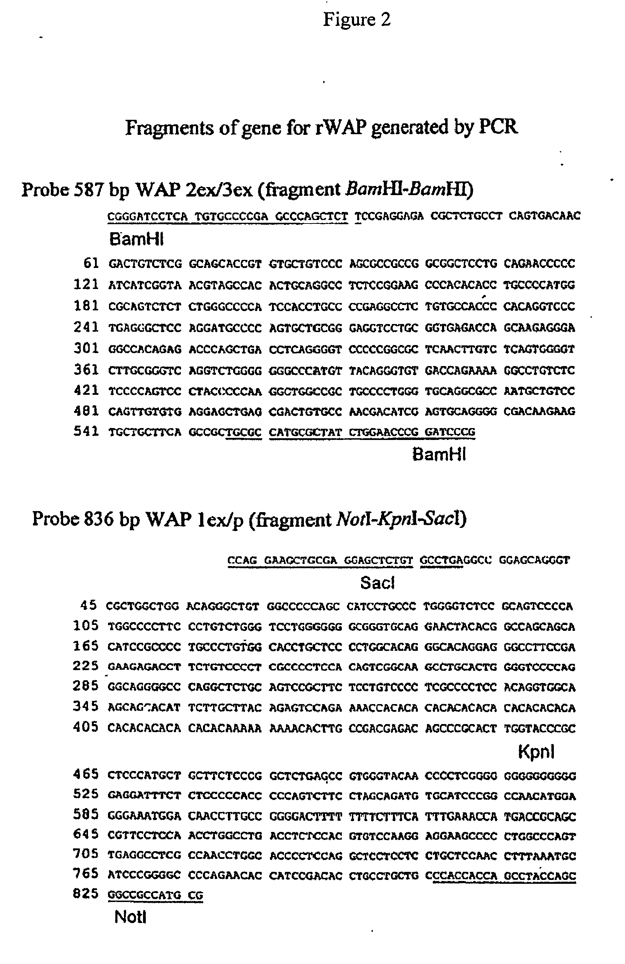

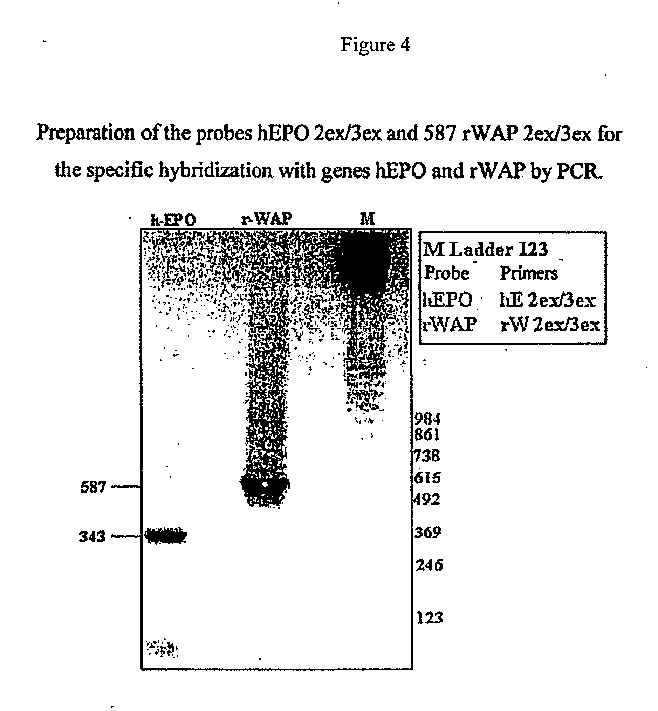

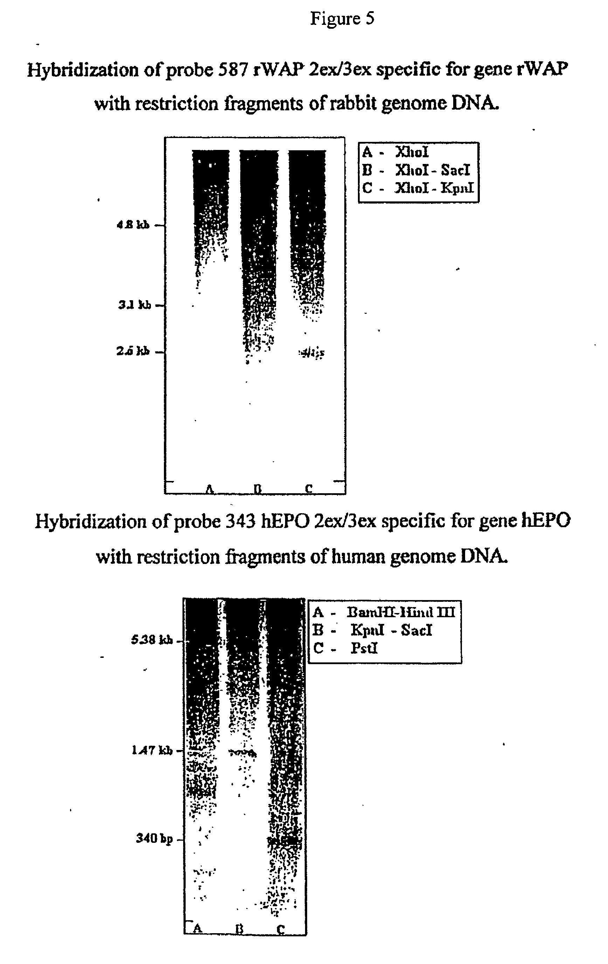

The rabbit WAP gene with the adjacent regulation regions was isolated from the DASH II phage genome rabbit library. The probe WAP 2ex/3ex 587 bp long (see Figure 2 and 4) was prepared by the polymerase chain reaction (PCR) on the genome DNA isolated from the rabbit muscle in a traditional way *(*Sambrook, J. Fritsch, E.F. Maniatis, T. (1989) Molecular cloning: laboratory manual -2nd ed. Cold. Spring. Harbor. Laboratory Press*.)* This DNA fragment is complementary to rabbit WAP gene sequence in the region between the second exon origin and the third exon end of the structural gene. These nucleotide primer sequences were used in PCR: 5'CGG GAT CCT CAT GTG CCC CGA GCC CAG CTC TT3'from the second exon origin of the WAP gene as a forward primer and 5'CGG GAT CCG GGT TCC AGA TAG CGC ATG GCG AC3'from third exon end of WAP as a reverse primer. Both primers were provided with the restriction site for BamHI restriction endonuclease at the 5'. Amplified fragment was isolated from the gel, digested with the restriction endonuclease BamHI and by ligation cloned into the vector pBluescript II KS+ (pBS). The ability of cloned DNA WAP gene fragment to specifically hybridize with the rabbit WAP locus was proved by Southern hybridization (see Figure 5). Genome DNA was digested with three pairs of restriction enzymes, separated on 0.7% m/v agarose gel, blotted and fixed on a nylon membrane. Hybridization with the labeled DNA fragment of WAP gene succeeded in detection of overall three fragments XhoI-XhoI 4.8 kb, XhoI-SacI 3.1 kb and XhoI-KpnI 2.6 kb.

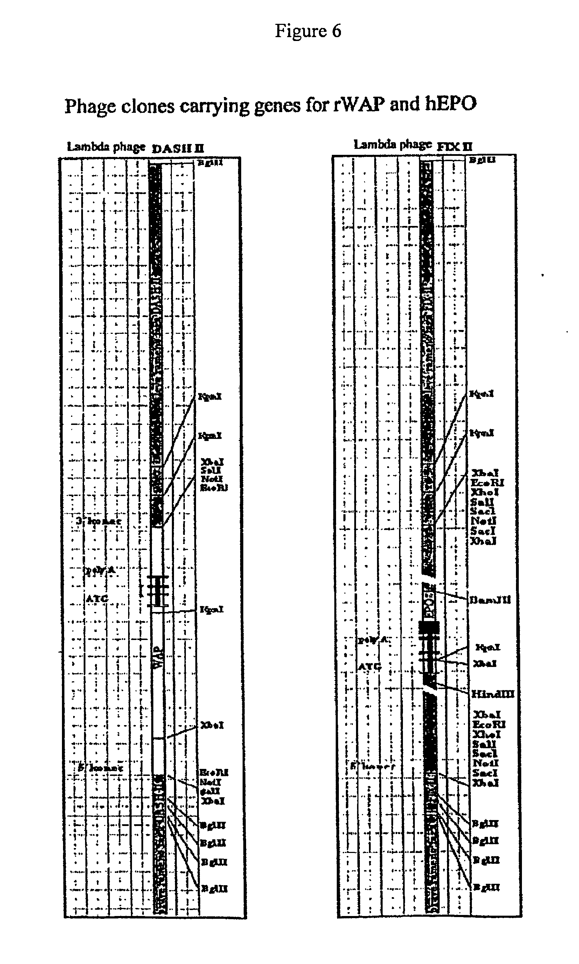

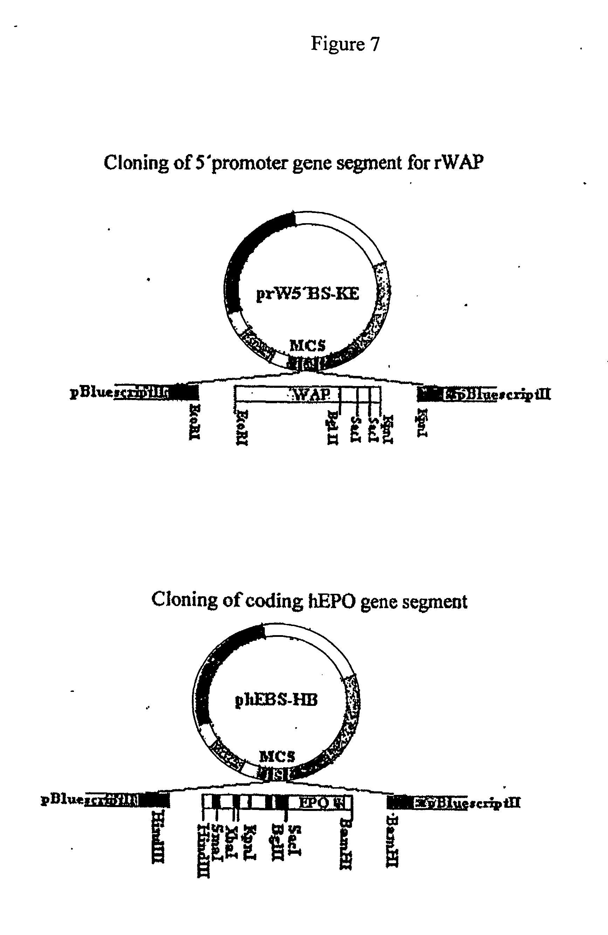

The proved probe was employed for detection of phage clones carrying WAP gene segments. From the plates an average of 150 mm across the plaques were printed on the nylon membranes and cultivated in the hybridization solution in standard conditions. This way the phage clone was successfully identified and isolated. This clone is carrying the full structure gene for WAP with adjacent 10 kb long 5'and and 4.8 kb long 3'sequences (see Figure 6). The 10 kb long 5' terminal fragment was isolated with the restriction enzymes EcoRI and KpnI on the 0.5% m/v agarose following the restriction analysis of λ phage DASH II containing a complete sequence of the rabbit WAP gene with its adjacent regulatory sequences. The fragment was cloned into the pBS vector and the resulting vector was labeled prW5'BS-KE (see Figure 7).

### Example 2: Sub-cloning of structural region of hEPO gene from FIXII phage genome library into plasmid phEBS-HB

Human structure gene for erythropoietin (EPO) was isolated from the FIX II phage genome human library.

The probe EPO 2ex/3ex 3343 bp long was prepared by PCR reaction on the genome DNA isolated from the human blood sample in a traditional way *(*Sambrook, J. Fritsch, E.F. Maniatis, T. (1989) Molecular cloning: laboratory manual -2nd ed. Cold. Spring. Harbor. Laboratory Press*.)* This DNA fragment is complementary to human EPO gene sequence in the region between the second exon origin and the third exon end of the structural gene (see Figure 4).

These nucleotide primer sequences were used in PCR: 5'TGT GAC AGC CGA GTC CTG CAG AGG3'from from the second exon origin of the EPO gene as a forward primer and 5'CAA GCT GCA GTG TTC AGC ACA GCC3' from third exon end of EPO as a reverse primer. Both primers were provided with the restriction site for PstI restriction endonuclease at the 5'. Amplified fragment was isolated from the gel, digested with the restriction endonuclease PstI and by ligation cloned into the vector pBS. The ability of cloned EPO fragment to specifically hybridize with the human EPO locus was proved by Southern blots (see Figure 5). Genome DNA was digested with three pairs of restriction enzymes, separated on 0.7% m/v agarose gel, blotted on a nylon membrane and hybridized with the radioactively labeled probe. In total three fragments BamHI- HindIII, SacI- SmaI and PstI were detected.

The proved probe was employed for detection of phage clones carrying EPO gene segments. From the plates an average of 150 mm across the plaques were printed on the nylon membranes and cultivated in the hybridization solution in standard conditions. This way the phage clone carrying the full structural gene for EPO was successfully identified and isolated (see Figure 6).

The 5.2 kb long fragment was isolated with the restriction enzymes BamHI and HindIII on the 0.5% m/v agarose following the restriction analysis of λ phage FIX II containing a complete sequence of the human EPO gene with its adjacent regulatory sequences. The fragment containing a complete human EPO gene sequence with its adjacent regulatory sequences was cloned into the pBS vector and the resulting vector was labeled phEBS-HB (see Figure 7).

### Example 3: Installation of NotI restriction site into the Kozak translational consensus of rWAP and hEPO genes

The selected strategy at the construction of hybrid genome segment between rWAP and hEPO is based on the target installation of a restriction site into the motif for Kozak translational consensus close to the start codon ATG. This target base modification is enabled by PCR technique with the terminally modified primers that are complementary at 3'-end with the target gene sequence and at 5'-end they carry the installed restriction site. The sequence with the restriction endonuclease *NotI* motif is attached to one of them (complementary to the Kozak translational consensus) using a primer pair. The region upstream from ATG gene rWAP with the modified Kozak translational consensus is generated via PCR. The modified hEPO gene region upstream from ATG is prepared in the same way mirror-wise. Both fragments are then combined through the modified translational consensus and interconnected with the remaining rWAP promoter regions and hEPO coding gene regions in the desired hybrid gene for targeted expression in the mammary gland cells.

The restriction analysis has proved that the *NotI* restriction site is not present in the region of the employed genome segments of EPO and WAP. Thus it is a suitable site for a construction of extensive recombinant vectors WAP/EPO.

The procedure of the *NotI* installation to Kozak sequence in WAP gene proceeded through the development of a synthetic WAP gene fragment by PCR using the following primers:
RW-832 (5' CCA ggA AgC TgC gAg gAg CtC TgT gCC TgA 3')
RW-26*NotI* (5' CgC Atg gCg gCCgCT ggT Agg CTg gTg gTg 3').

The modified fragment of EPO gene with *NotI* site in Kozak sequence was created by PCR with this primer pair:
hE+1*NotI* (5' Aag Aat gCg gCC gCC Atg ggg gTg CaC ggT gAg TAC TCg 3')
hE+984 (5' CAA gCT gCA gTg TTC AgC ACA gCC 3').

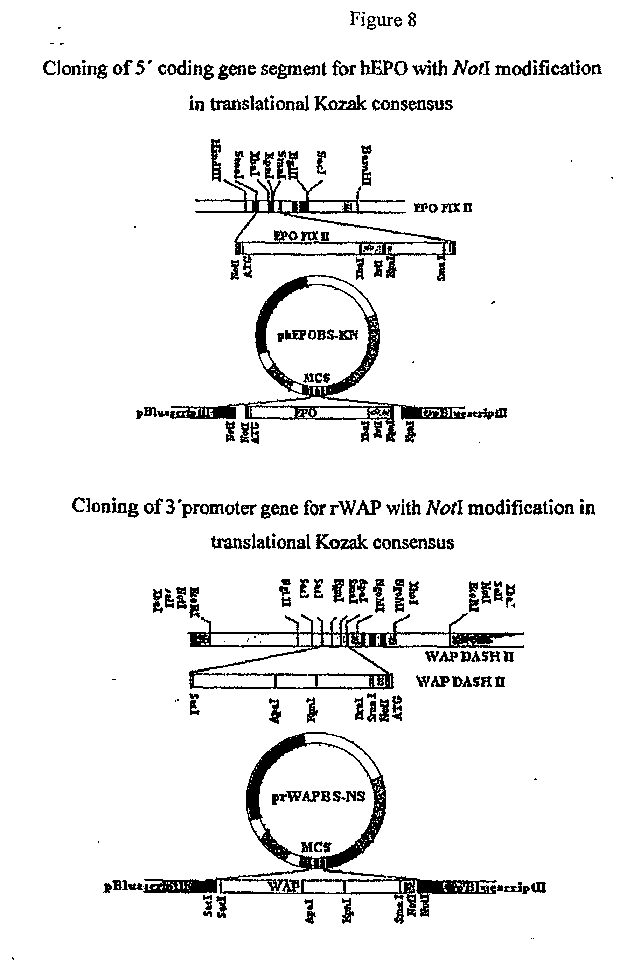

Both amplified fragments were isolated from gel, tested by restriction enzymes and modified for ligation: WAP fragment was digested with *NotI* and *SacI* enzymes, EPO fragment was digested by *NotI* and *KpnI* pair. *NotI-* modified EPO fragment was cloned to the host operative vector pBS under creation of the vector phEPOBS-KN (see Figure 8). The modified WAP fragment was also subcloned to the same host vector and the construct was labeled as vector prWAPBS-NS (see Figure 8).

### Example 4: Cloning and ligation of rWAP and hEPO gene fragments with installed NotI restriction site

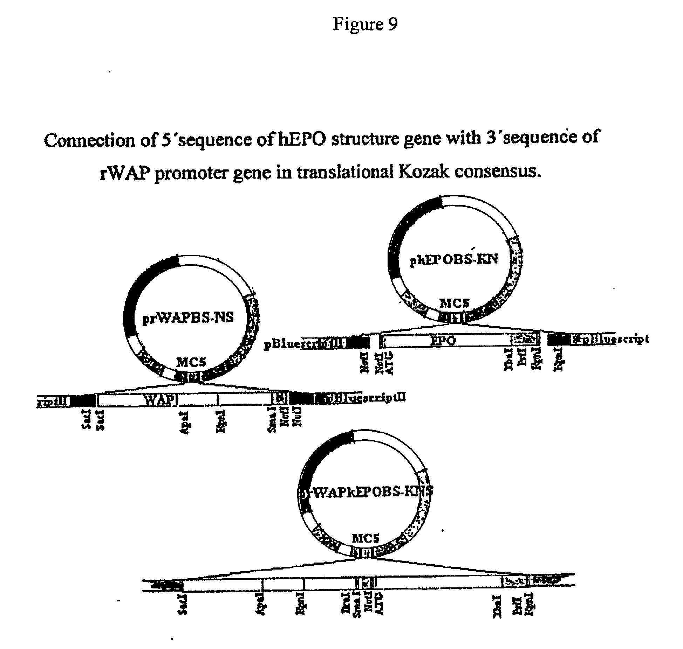

PCR-generated *NotI*-modified rWAP gene fragment was installed to vector phEPOBS-KN through the newly created *NotI* site in EPO Kozak-sequence and through *SacI* site in the vector polylinker. Vector was labeled as prWAPhEPOBS-KNS (see Figure 9). The resulting WAP/EPO fusion fragment *SacI- (NotI)-KpnI* of the size 1070 bp gave the base to the development of the targeting hybrid construct.

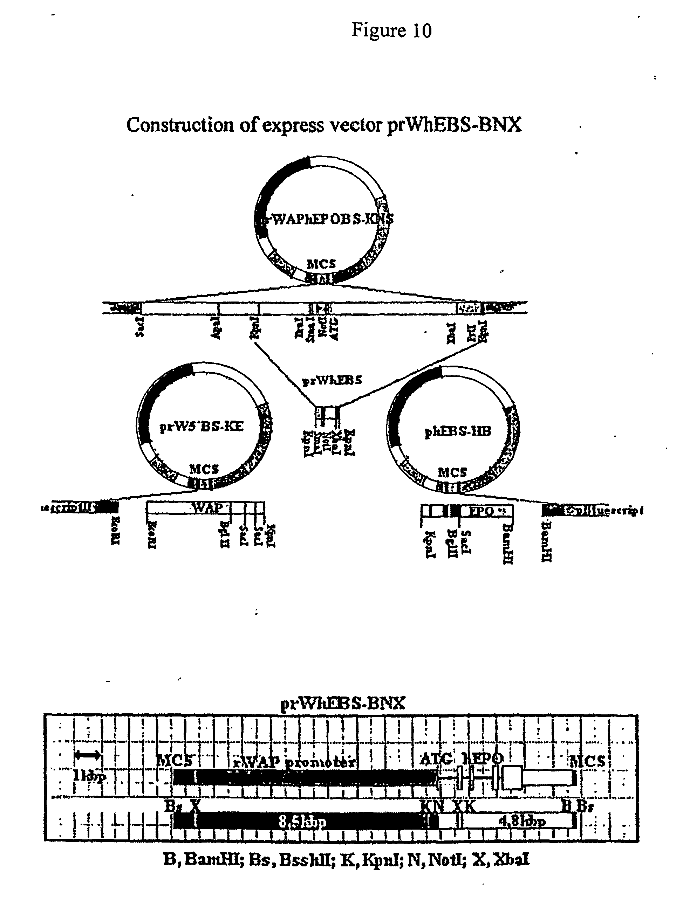

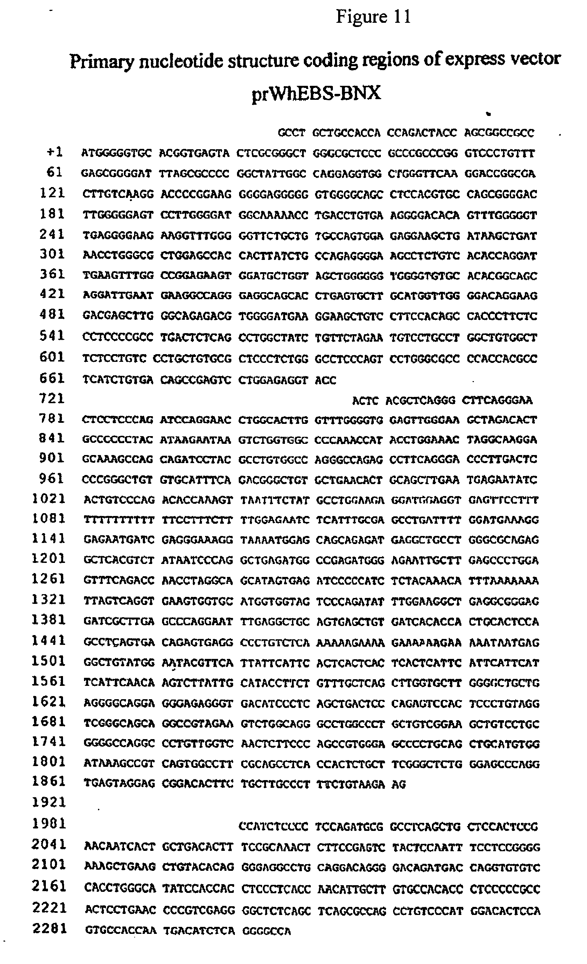

Vector prWBS-KE, carrying the subcloned 5'-nontranslated regulatory region of the rabbit WAP gene was linearized with *KpnI* enzyme at site-383 from ATG start and subsequently ligated with *Kpn*I*-*(*Not*I)*-Kpn*I 1070 bp long connecting fragment isolated from prWAPhEPOBS-KNS. The fragment fusion created vector prWhEPOBS-KNKE 14 kb long. Vector phEBS-HB carrying the entire coding region of human EPO gene, was linearized with *KpnI* enzyme at site +687 from ATG start and subsequently ligated with *Kpn- (NotI)-KpnI* 1070 bp long connecting fragment isolated from prWAPhEPOBS-KNS. The fragment fusion created vector prWAPhEBS-KNKB 9 kb long. *NotI-BamHI* fragment 4.8 bp long was subsequently cleaved out from this vector. The fragment contained the complete structural gene for hEPO with the installed *NotI* site in Kozak sequence and it was inserted into the host vector pBS under formation of phEBS-BN. The *XbaI-XbaI* fragment 8kb big isolated from the prWhEPOBS-KNKE vector was subsequently inserted to the *XbaI* site of this construct. The resulting recombinant construct, called prWhEBS-BNX (see Figure 10) was tested by restriction enzymes and partially sequenced (see Figure 11). The size of the entire vector is 16 kb and the hybrid rWAP-hEPO gene can be isolated from the vector by digesting with the enzymes *EcoRI* and *HindIII.*

### Identification of transgenic mice

The system of the primers previously exploited to prepare the probes whose specificity was proved by hybridization with a human genome DNA, was tested for an identification of transgenic mice. It was proved that the tested primers on negative murine DNA do not allow PCR amplification of any fragment.

### Industrial applicability

The way of modification of the translational Kozak consensus rWAP gene according to the invention is exploitable for an easy construction of expression vectors specified for transgenic protein production. The vector prWhEBS-BNX prepared by the described technique can be used for EPO expression in the suitable transfected eukaryotic cells or in the mammary gland of transgenic organisms.

## Claims

1. Method for the preparation of the expression vector for therapeutic protein production **characterized in that** a nucleic acid molecule comprising a sequence motif GCGGCCGCCATGG for restriction endonuclease Notl within the region from nucleotides -9 to +4 of translational Kozak sequence, related to the starting ATG codon, is inserted into a vector molecule such that said nucleic acid molecule connects a gene fragment comprising a 5'end non-translated regulatory promoter sequence including said translation Kozak sequence with a gene fragment comprising coding sequence of said therapeutic protein as shown in Fig. 1.

2. Method for the preparation of a nucleic acid expression vector according to claim 1, **characterized in that** said gene fragment comprising a promoter regulatory region is an 8.5 kb long 5'end non-translated regulatory promoter sequence including said translation Kozak sequence and is derived from the rabbit whey acidic protein gene and wherein said gene fragment contained in said translation Kozak sequence comprises a coding sequence of the human erythropoietin.

3. A nucleic acid expression vector **characterized in that** it comprises the nucleic acid sequences as shown in Fig. 11 obtainable by any method according to claims 1 and 2.

4. A eukaryotic cell, with the exception of human embryonic stem cell, transformed with a nucleic acid expression vector according to claim 3.

5. A non-human transgenic organism transformed with a nucleic acid expression vector according to claim 3.

## Patentansprüche

1. Methode für die Präparation des Expressionsvektors für die Produktion von einem therapeutischen Protein, **dadurch gekennzeichnet, dass** ein Nukleinsäuremolekül, das ein CCGGCCGCCATGG Sequenzmotiv für die Notl-Restriktionsendonuklease innerhalb der Region von dem -9 bis zu dem +4 Nukleotiden der translationalen Kozak-Sequenz, die mit dem Ausgangs-ATG-Codon verwandt ist, aufweist, in ein Vektormolekül eingefügt wird, so dass das genante Nukleinsäuremolekül, ein Genfragment, das eine 5'Ende nicht translationierte regulatorische die genannte Translation-Kozak-Sequenz einschließende Promotorsequenz aufweist, mit einem Genfragment, das eine Kodierungssequenz des genannten therapeutischen Proteins, wie es in der Abb. 1 abgebildet ist, verbindet.

2. Methode für die Präparation eines Expressionsvektors einer Nukleinsäure nach Anspruch 1, **dadurch gekennzeichnet, dass** das genannte eine regulatorische Promotorregion aufweisende Genfragment eine 8,5 kb lange 5'Ende nicht translationierte regulatorische die genannte Translation-Kozak-Sequenz einschließende Promotorsequenz ist und aus dem Säureprotein der Kaninchenmolke abgeleitet ist und wobei das genannte in der Translation-Kozak-Sequenz umgefasste Genfragment eine Kodierungssequenz des menschlichen erythropoletin aufweist.

3. Ein Expressionsvektor der Nukleinsäure, **dadurch gekennzeichnet, dass** er Sequenzen der Nukleinsäure, wie sie in der Abb. 11 abgebiltet sind, umfasst, die durch das Verfahren nach Ansprüchen 1 und 2 erhältlich sind.

4. Eine eukaryotische Zelle, mit der Ausnahme der humanen embryonalen Stammzelle, die mit einem Expressionsvektor einer Nukleinsäure nach Anspruch 3 transformiert ist.

5. Ein nicht-menschliches transgenes Organismus, das mit einem Expressionsvektor einer Nukleinsäure nach Anspruch 3 transformiert ist.

## Revendications

1. Méthode pour la préparation d'un vecteur d'expression pour une production de la protéine thérapeutique **caractérisée en ce que** l'ADN molécule comprenant la séquence GCGGCCGCATGG pour l'endonucléase de réstriction NotI dans la région des nucléotides de -9 à +4 de la séquence de Kozak par rappport au codon d'initiation ATG, est insérée dans la molécule de vecteur de façon que ladite molécule d'acide nucléique connecte un fragment du gène comprenant une séquence régulatrice non-traduite de promoteur de l'extrémité 5', comprenant ladite séquence de Kozak, avec le fragment du gène comprenant une séquence codant pour ladite protéine thérapeutique comme le montre la figure 1.

2. Méthode pour la préparation de l'ADN vecteur d'expression selon la revendication 1 **caractérisée en ce que** ledit fragment du gène comprenant une région régulatrice de promoteur est une séquence régulatrice non-traduite de promoteur de l'extrémité 5', longue de 8,5 kb comprenant ladite séquence de Kozak et est dérivé du gène pour la protéine WAP (whey acidic protein) de lapin et où ledit fragment du gène compris dans ladite séquence de Kozak comprend une séquence codant de l'erythropoïétine humaine.

3. L'ADN vecteur d'expression **caractérisé en ce qu'**il comprend les séquences d'acides nucléiques montrées dans la figure 11 obtenables par une méthode quelconque selon l'une des revendications 1 et 2.

4. Une cellule eucaryote à l'exception de la cellule souche embryonnaire humaine transformée avec l'ADN vecteur d expression selon la revendication 3.

5. L'organisme transgénique non humain transformé avec l'ADN vecteur d'expression celon la revendication 3.
